# EUROPEAN PATENT APPLICATION

(11) **EP 4 000 614 A1**
(43) Date of publication of application: **25.05.2022**
(21) Application number: 20382996.5
(22) Date of filing: 18.11.2020
(51) Int. Cl.: A61K 31/352, A61P 31/14, A61K 45/06

(54) **EFLOXATE FOR USE AS ANTIVIRAL AGENT**

(71) Applicant: Consejo Superior de Investigaciones Científicas (CSIC), 28006 Madrid (ES)
(72) Inventor: MARTINEZ GIL, Ana, 28040 Madrid (ES); GIL AYUSO-GONTAN, Carmen, 28040 Madrid (ES); CAMPILLO MARTIN, Nuria, 28040 Madrid (ES); GASTAMINZA LANDART, Pablo, 28049 Madrid (ES); GARAIGORTA DE DIOS, Urtzi, 28049 Madrid (ES)
(74) Representative: Pons

(57) **Abstract**

The present invention relates to new use of efloxate. In particular it refers to its use as antiviral agent.

## Description

The invention relates to a new use of efloxate. In particular, it refers to efloxate or a pharmaceutically acceptable salt, tautomer and/or solvate thereof for use as antiviral agent.

Therefore, the present invention belongs to the field of medicine.

### BACKGROUND ART

Viruses, which cause infectious disease in humans and other mammals, are a diverse group of infectious agents that differ greatly in size, shape, chemical composition, host range, and effects on hosts. A variety of antiviral agents are available for the treatment and/or prevention of diseases caused by viruses, such as hepatitis B, influenza A and B and HIV, etc. However, some known viruses and the emergence of new viruses that are immune to currently available antiviral agents make new and effective antiviral agents necessary. In particular, among the known viruses, coronaviruses (CoVs) act as cross-species viruses and have the potential to spread rapidly into new host species and cause epidemic diseases. Despite the severe public health threat of severe acute respiratory syndrome coronavirus (SARS-CoV) and Middle East respiratory syndrome coronavirus (MERS-CoV), there are currently no effective drugs available for their treatment; therefore, broad-spectrum inhibitors of emerging and endemic CoVs are urgently needed.

Moreover, since the emergence of a novel coronavirus (SARS-CoV-2) from Wuhan, China, in December 2019, that has caused millions of cases of human infections and huge cases of deaths globally and was declared as a pandemic by World Health Organisation, broad-spectrum inhibitors of emerging and endemic CoVs are needed. The virus spreads rapidly among humans, and the symptoms caused by the virus (abbreviated "COVID-19") ranges from mild coughs and fevers to severe pneumonia and potentially life-threatening symptoms.

Waiting for the availability of new vaccines, this global emergency has triggered the search for safe and effective pharmacological approaches for eradicating the virus, or at least reducing its effects and hindering the contagion, since, currently there is no effective therapy to treat SARS-CoV-2 infection.

Accordingly, there is a need for safe and effective antiviral agents with a wide-spectrum of anti-viral activity, especially against coronaviruses.

In view of this need, the present invention provides an already available drug that so far has not been used as antiviral and more particularly against coronaviruses infection.

### SUMMARY OF THE INVENTION

The present invention in based on the surprising finding that efloxate may be used to prevent or/and treat a virus infection.

The inventors have found a consistent antiviral activity of efloxate in cells infected by coronavirus, in concentrations of the drug that were non-cytotoxic.

The compound efloxate has the following formula:

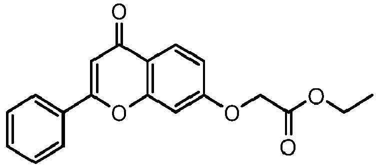

It is a known vasodilator, used to treat chronic coronary insufficiency and Angina pectoris. This drug is readily available to the public and can be purchased from chemical companies.

Then, the present invention relates to efloxate or a pharmaceutically acceptable salt, tautomer and/or solvate thereof, for use in the prevention and/or the treatment of virus infection, that is, as antiviral agent.

In a preferred embodiment, the present invention refers to efloxate or a pharmaceutically acceptable salt, tautomer and/or solvate thereof for use in the prevention and/or the treatment of coronaviruses infection. More particularly in the prevention and/or the treatment of human coronavirus 229E infection or coronavirus SARS-CoV-2 infection.

Unless otherwise stated, the compounds used in the invention are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

The compounds used in the invention may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

For its application in therapy, efloxate or a pharmaceutically acceptable salt, tautomer and/or solvate thereof will preferably be in a pharmaceutically acceptable or substantially pure form, that is, with a pharmaceutically acceptable level of purity excluding pharmaceutically acceptable excipients and not including material considered toxic at normal dosage levels. The purity levels for a compound of formula (I) are preferably above 50%, more preferably above 70%, more preferably above 90%. In a preferred embodiment, they are above 95%.

The term pharmaceutically acceptable salt embraces salts with a pharmaceutically acceptable acid or base.

The term "pharmaceutically acceptable" relates to molecular entities and compositions that are physiologically tolerable and do not typically produce an allergic reaction or a similar unfavourable reaction, such as gastric upset, dizziness and similar side effects, when administered to a subject. Preferably, the term "pharmaceutically acceptable" means approved by a regulatory agency of a federal or state government or collected in the US Pharmacopoeia or other generally recognised pharmacopeia for use in animals and, more particularly, in humans.

"Tautomers" are understood to be the two isomers that differ only in the position of a functional group because between the two forms there is a chemical balance in which a migration of a group or atom occurs.

The term "treatment or prevention" as used herein, unless otherwise indicated, relates to reversing, alleviating and inhibiting the progress of, or preventing the disorder or condition to which it applies in such terms, one or more symptoms of such disorder or condition.

Other aspect of the present invention relates to a composition comprising efloxate or a pharmaceutically acceptable salt, tautomer and/or solvate thereof for use in the prevention and/or the treatment of virus infection.

In particular, the composition of the invention is a pharmaceutical composition. The term "pharmaceutical composition" means the composition that includes efloxate or a pharmaceutically acceptable salt, tautomer and/or solvate thereof and at least one excipient, adjuvant and/or pharmaceutically acceptable carrier.

The term "excipients, adjuvants and/or carriers" relates to molecular entities or substances through which the active ingredient is administered. Such pharmaceutical excipients, adjuvants or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes.

Efloxate or a pharmaceutically acceptable salt, tautomer and/or solvate thereof or the pharmaceutical composition comprising them can be administered by any suitable administration route, for example, oral, topical, parenteral or rectal administration. It is administrated in a therapeutically effective amount.

In a particular embodiment, said pharmaceutical compositions may be in a pharmaceutical form of oral administration, either solid or liquid. Illustrative examples of pharmaceutical forms of oral administration include tablets, capsules, granules, solutions, suspensions, etc., and may contain conventional excipients such as binders, dilutes, disintegrating agents, lubricants, humectants, etc., and may be prepared by conventional methods. The pharmaceutical compositions may also be adapted for parenteral administration, in the form of, for example, solutions, suspensions or lyophilised, sterile products in the suitable dosage form; in this case, said pharmaceutical compositions will include suitable excipients, such as buffers, surfactants, etc. In any case, the excipients are chosen according to the pharmaceutical form of administration selected.

As used herein, the term "therapeutically effective amount" means the necessary amount of a compound for the treatment or prevention of the disease, disorder or condition to be effective. It will also depend on the subject to be treated, the severity of the illness suffered by said subject, the chosen form of administration, etc. For this reason, the doses mentioned in this invention should be considered only as guidelines for the person skilled in the art, and should be adjusted the doses according to the variables mentioned above

Efloxate or a pharmaceutically acceptable salt, tautomer and/or solvate thereof as well as the compositions containing them can be used in conjunction with other additional antiviral drugs useful in the prevention and / or treatment of diseases caused by virus infections. Said additional drugs may form part of the same composition or, alternatively, may be provided in the form of a separate composition for simultaneous or sequential administration to efloxate or a pharmaceutically acceptable salt, tautomer and/or solvate thereof.

In another aspect, the invention relates to a method for the prevention and/or treatment of virus infection in a subject, comprising the administration to said subject of a therapeutically effective amount of efloxate or a pharmaceutically acceptable salt, tautomer and/or solvate thereof.

As used in this description, the term "subject" includes any animal or human bring. Preferably the subject is a human being.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to be limiting of the present invention.

### EXAMPLES

Some examples carried out by the inventors are provided in order to illustrate the invention.

### Example 1. Human Coronavirus 229E-GFP infection assays

Huh7-Lunet#3 cells (hepatoma cell line; kindly provided by Dr. Thommas Pietschmann; Twincore-Hannover) were maintained subconfluent in complete media [(DMEM supplemented with 10 mM HEPES, 1X non-essential amino acids (Gibco), 100 U/ mL penicillin-streptomycin (Gibco) and 10 % Fetal Bovine Serum (FBS; heat-inactivated at 56 °C for 30 minutes)]. Recombinant 229E-GFP virus (kindly provided by Dr. Volker Thiel; University of Bern and described in Cervantes-Barragan L, et al. Dendritic cell-specific antigen delivery by coronavirus vaccine vectors induces long-lasting protective antiviral and antitumor immunity. mBio. 2010 Sep 14;1(4). pii: e00171-10. doi: 10.1128/mBio.00171-10) is a surrogate model of human 229E coronavirus infection that expresses green fluorescent protein as a marker of infection.
Huh7-Lunet#3 cells were seeded onto 96-well plates (1x10⁴ cells/well). The day after, compound (Efloxate, MicroSource Discovery System Inc) stock solutions (10 mM in DMSO) were diluted into complete cell culture media to achieve a final concentration of 100 µM. The 100 µM solution was serially diluted 3-fold to achieve decreasing compound concentrations. On the other hand, 229E-GFP virus stock was diluted in complete media to achieve a final concentration of 3x10³ focus forming units (FFU)/mL. One hundred microliters (100 µL) of the virus dilution were mixed 1:1 with 100 µL of the Efloxate dilutions to achieve final compound concentrations in a range from 50 µM to 22 nM and 150 infectious units (FFU) per well in a 96 well plate. One hundred µl of the mixture was applied onto the Huh7-Lunet #3 cell monolayer in biological replicates and cells were cultured for 72 hours at 33°C in a 5% CO₂ incubator. Cells were fixed in a 4% formaldehyde solution in FBS for 10 minutes at room temperature, washed twice with FBS and individual well fluorescence was measured in a SpectraMax iD3 fluorescence plate reader (Molecular Devices). Background subtraction was performed using non-infected wells and signal was normalized to the average fluorescence found in vehicle (DMSO)-treated virus-infected wells. Relative infection efficiency was plotted versus compound concentration to determine the EC₅₀ and EC₉₀ (50% and 90% maximal effective concentration, respectively) values. Once infection efficiency had been determined, plates were stained with a 0.1% crystal violet solution in water-methanol for 30-60 minutes. Then, plates were extensively washed with water and dried before 1% SDS solution in water was added to solubilize crystal violet. Absorbance was measured at 570 nm and background was subtracted from blank wells. Relative well biomass was estimated by calculating the absorbance in each well relative to the average observed in infected cells treated with DMSO. Relative biomass was plotted versus efloxate concentration to determine the cytotoxic concentration (CC₅₀) values.
Data are collected in table 1.

**Table 1: Efloxate Potency and cytotoxicity indexes in coronavirus 229E-GFP infection assays**

| | **229E-GFP** | | |
|---|---|---|---|
| Drug name | EC₅₀ (µM) | EC₉₀ (µM) | CC₅₀ (µM) |
| Efloxate | 1.6 | 5 | 35 |

| | | | |
|---|---|---|---|
| Efloxate shows efficacy to block the 90% of 229E-GFP viral infection at concentrations below the cytotoxic one. Thus, efloxate behaves as an antiviral agent against human coronavirus 229E-GFP. | | | |

### Example 2. SARS-CoV-2 infection assays

All infection experiments were performed by inoculating Vero-E6 cells (ATCC CRL-1586^{™}) seeded onto 96-well plates (2x10⁴ cells/well) with the SARS-CoV-2 strain NL/2020 (kindly provided by Dr. R. Molenkamp, Erasmus University Medical Center Rotterdam) at low multiplicity of infection (MOI) of 0.001. Cultures were maintained at 37°C in a 5% CO₂ incubator for different lengths of time depending on the experiment. Efloxate was diluted from 10 mM stock solutions in complete media containing 2% FBS to achieve the indicated final concentrations.
Cell monolayer protection assays: Vero-E6 cell monolayers were inoculated at MOI 0.001 in the presence of a wide range of compound concentrations (from 50 to 0.78 µM) of Efloxate in duplicate wells. Seventy-two hours later the cells were fixed using a 4% formaldehyde-PBS solution to finalize the experiment and inactivate the virus. Virus-induced cell death was revealed by staining with a 0.1% crystal violet solution in water-methanol for 30-60 minutes, time after which, plates were extensively washed with water and dried. Infected, as well as uninfected cells, were used as controls. Using crystal violet staining, the inventors determined the maximum and minimum protective concentration (PCmax and PCmin) as indicated above (Table 2).

Cytotoxicity measurement by MTT assays (MTT assay: cell viability assay that infers reductive capacity of the cells via formation of formazan precipitates. Overall estimation of cell metabolic rate): Vero-E6 cell monolayers were treated with a wide range of compound concentrations (from 50 to 0.78 µM) and forty-eight hours later they were subjected to MTT assays following the manufacturer's instructions. Compound concentration leading to a 50% of cytotoxicity (CC₅₀) were determined (Table 2).

Intracellular viral RNA quantitation: To confirm that protection of the monolayer was indeed due to the ability of efloxate to restrict virus replication, viral RNA quantitation was performed as indicated below. Vero-E6 cell monolayers were inoculated at MOI 0.001 in the presence of the indicated compound concentrations (dose µM). Forty-eight hours later cell lysates were prepared using Trizol reagent (Thermo Scientific). Viral RNA content was determined by RT-qPCR using previously validated sets of primers and probes specific for the detection of the SARS-CoV-2 E RNA [Corman VM et al. Euro Surveill., 2020, 25:2431] and the cellular 28S RNA, for normalization purposes. ΔCt method was used for relative quantitation of the intracellular viral RNA accumulation in compound-treated cells compared to the levels in infected cells treated with DMSO, set as 100%. Log drop, i. e. the relative reduction in viral RNA accumulation in log scale, for compound are collected in Table 2.

**Table 2: Efloxate Potency and cytotoxicity indexes in coronavirus SARS-CoV-2 infection assays**

| **PC MAX (µM)** | **PC MIN (µM)** | **HIGHEST NON-TOXIC CONCENTRATION (MTT)** | **DOSE (µM) TESTED in VIRAL LOAD** | **VIRAL LOAD MOI 0.001 (% OF CONTROL)** | **LOG DROP** | **MTT CC₅₀ (µM)** |
|---|---|---|---|---|---|---|
| 50 | 25 | 12.5 | 50 | 0.06 | 3.2 | >50 |
| | | | 35 | 0.00 | 5.5 | |
| | | | 25 | 24.34 | 0.6 | |
| | | | 12.5 | 27.42 | 0.55 | |

| | | | | | | |
|---|---|---|---|---|---|---|
| PC Max: Maximum concentration of compound that protects cells (100%) from the cytopathic effect induced by the virus. PC Min: Minimum concentration of compound that protects cells (100%) from the cytopathic effect induced by the virus. MTT CC₅₀: Concentration of compound that causes a 50% reduction in the MTT assay | | | | | | |

### Example 3. Evaluation of anti-SARS-CoV-2 drug as entry inhibitor

As viral mutations represent one of the main challenges to overcome with antiviral therapies, the inventors have carried out a multitarget virtual screening focused on druggable targets related to viral entry followed by biological screening against SARS-CoV-2 to identify host-directed therapies against COVID-19. They have considered mainly host-based proteins involved in SARS-CoV-2 entry, together with the interaction between the external viral spike protein and the human ACE2 receptor. The US Drug Collection of 1798 compounds of FDA approved drugs has been screened toward the proteases, TMPRSS2, Furin and Cathepsin L, the kinases AAK1, GAK and PIKfyve and the two-pore ion channel TPC2, all of them with a known role in the virus entry process. Moreover, the complex between the receptor binding domain for the SARS-CoV-2 Spike (S) glycoprotein, key viral component for the entry process, and human ACE2 has been included in the analysis. As a confirmation of the effectiveness of the method, efloxate has been confirmed as new antiviral agent.

Because the SARS-CoV-2 inhibitor described above has been identified by a multitarget host-based entry targets screening, its ability to interfere with entry of SARS-CoV-2 S protein-pseudotyped retroviral vectors was evaluated. This system consists in the production of reporter retroviral vectors pseudotyped with the envelope glycoprotein S (Spp) which is a major determinant of SARS-CoV-2 entry. This system enables evaluation of virus entry infection efficiency as a function of the reporter gene activity (luciferase), which is strictly dependent on the presence of a functional viral glycoprotein. As a control, pseudotypes based on vesicular stomatitis virus (VSV-G) glycoprotein were studied in parallel to determine the selectivity of the antiviral candidates. VSV-G pseudotypes use the endocytic pathway to enter the cells, although through a different receptor that S-pseudotypes. On the other hand, RD114-pseudotypes, also used in this study, are internalized after direct fusion of the viral envelope with the cell plasma membrane.
Production of retroviral particles pseudotyped with different viral envelopes has previously been described. Packaging plasmids, vesicular stomatitis virus (VSV) G and RD114 glycoprotein expressing plasmids were kindly provided by Dr. F. L. Cosset (INSERM, Lyon). SARS-CoV-2 S expressing plasmid was obtained from Jose Maria Casanovas and Juan Garcia Arriaza (CNB-CSIC). Particles devoid of envelope glycoproteins were produced in parallel as controls.
For SARS-CoV-2 Spike pseudotyped particle (SARS2pp) entry experiments, Vero-E6 cells (10⁴ cells/well) were seeded onto 96-well plates the day before. Compounds were diluted in complete media [(DMEM supplemented with 10 mM HEPES, 1x non-essential amino acids (Gibco), 100 U/ mL penicillin-streptomycin (Gibco) and 10% Fetal Bovine Serum (heat-inactivated at 56 °C for 30 min)] to achieve a 2x concentration. Fifty microliters (50 µL) of the SARS-CoV-2 Spp, VSVpp or RD114pp retrovirus dilutions were mixed 1:1 with 50 µL of the 2x compound dilutions to achieve the desired final compound concentrations, as indicated in the figure. One hundred µL of the mixture was applied onto the Vero E6 cell monolayer in biological triplicates and cells were cultured at 37 °C in a 5% CO₂ incubator. Forty-eight hours post-inoculation, cells were lysed for luciferase activity determination using Luciferase Assay System (Promega) and a luminometer. Relative infection values were determined by normalizing the data to the average relative light units detected in the vehicle control cells. Data are recorded in table 3.

**Table 3. Pseudotyped retroviral vectors entry efficiency of efloxate**

| Drug | Dose | Spp (% of control) | VSVpp (% of control) | RD114pp (% of control) |
|---|---|---|---|---|
| Efloxate | 50 µM | 16.68 | 106.24 | 153.15 |

Analysis of the pseudotype entry efficiency shows that efloxate interfere selectively with Spp entry, as predicted by the potential endocytic targets identified in the virtual screening.

According to these results, efloxate presents antiviral activity in human coronavirus, including SARS-CoV-2 at non-cytotoxic concentrations (12.5-50 µM) showing potential to be used as treatment for COVID-19. Moreover, as its mechanism of action involved viral endocytic entry targeting host targets, efloxate may be also active in other highly pathogenic virus such is the case of Ebola, Zika or Crimea-Congo viruses.

## Claims

1. Efloxate, a pharmaceutically acceptable salt, tautomer and/or solvate thereof or a composition comprising it for use as antiviral.

2. Efloxate, a pharmaceutically acceptable salt, tautomer and/or solvate thereof or a composition comprising it for use, according to claim 1, in the prevention and/or the treatment of coronavirus infection.

3. Efloxate, a pharmaceutically acceptable salt, tautomer and/or solvate thereof or a composition comprising it for use, according to claim 2, in the prevention and/or the treatment of human coronavirus 229E infection.

4. Efloxate, a pharmaceutically acceptable salt, tautomer and/or solvate thereof or a composition comprising it for use, according to claim 2, in the prevention and/or the treatment of human coronavirus SARS-CoV-2 infection.

5. Efloxate, a pharmaceutically acceptable salt, tautomer and/or solvate thereof or a composition comprising it for use, according to any of previous claims, in combination with another antiviral agent.

6. Efloxate, a pharmaceutically acceptable salt, tautomer and/or solvate thereof or a composition comprising it for use, according to any of previous claims, wherein its administration is oral, topical, parenteral or rectal.
